# EUROPEAN PATENT SPECIFICATION

(11) **EP 2 548 594 B1**
(45) Date of publication and mention of the grant of the patent: **12.07.2023**
(21) Application number: 11006015.9
(22) Date of filing: 22.07.2011
(51) Int. Cl.: A61K 9/00, A61M 5/315, A61K 9/08, A61K 31/197, A61P 21/02

(54) **Intrathecal baclofen pharmaceutical dosage forms and related delivery system**
Intrathekale pharmazeutische Baclofen-Dosisformen und zugehöriges Abgabesystem
Formes de dosage pharmaceutiques de baclofène intrathécal et système de distribution associé

(43) Date of publication of application: 23.01.2013
(73) Proprietor: Piramal Critical Care Inc., Bethlehem, PA 18017 (US)
(72) Inventor: Foster, John, Woodbury, MN 55125 (US); Prentice, Thomas R., Woodbury, MN 55125 (US)
(74) Representative: HGF

(56) References cited:
- WO-A1-2007/035621
- WO-A2-2011/163647
- US-A1- 2005 004 219
- US-A1- 2010 056 989
- US-A1- 2010 106 097
- US-B1- 6 629 954

## Description

The present invention relates generally to a syringe or vial that is filled in advance with a liquid to be injected. More specifically, the present invention is directed to pre-filled syringes and vials to fill and refill infusion systems with existing and new forms of intrathecal baclofen.
In an aspect, the present invention provides an intrathecal drug delivery system comprising:
(a) a sterilised syringe (10) with a leur-lock tip (13); characterised in that
(b) the sterilised syringe containing a sterile, stable solution consisting of baclofen, sodium chloride and water which has been formed aseptically and terminally sterilized and inserted into the sterilized syringe with minimal head space;
(c) a color coding system on a label or plunger of the sterilised syringe indicative of various concentrations of baclofen of (b) or indicative of size of the sterilised syringe; and
(d) instructions for use.

Baclofen is a skeletal muscle relaxant and antispastic agent. Baclofen is a structural analog of the inhibitory neurotransmitter gamma-aminobutyric acid (GABA), and may exert its effects by stimulation of the GABA_{B} receptor subtype. Baclofen is the generic (USAN) name (USP Dictionary of USAN and International Drug Names 2003) for 4-amino-3-(p-chlorophenyl) butyric acid, a derivative of γ-aminobutyric acid. Its structural formula is:

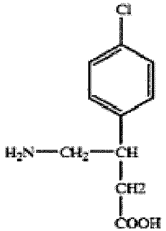

Baclofen is a white to off-white, odorless or practically odorless crystalline powder, with a molecular weight of 213.66 g/mol. It is slightly soluble in water, very slightly soluble in methanol, and insoluble in chloroform. Baclofen can be administered orally, but when injected directly into the intrathecal space of a patient effective cerebrospinal fluid (CSF) concentrations are achieved with resultant plasma concentrations 100 times less than those occurring with oral administrations.

As indicated in US patent number 7,824,697, baclofen solutions having concentrations in the range of about 3 to about 8 mg/mL can be obtained by mixing the appropriate quantity of baclofen with an aqueous diluent and heating the solution to a temperature of at least about 30° C., at least about 40° C., at least about 50° C., preferably at least about 60° C., and most preferably at least about 70° C. and a temperature of less than about 90° C., less than about 95° C., less than about 100° C., less than about 121° C., or most preferably less than the temperature at which baclofen thermal degrades to a significant degree. The heat is applied while simultaneously subjecting the solution to intense agitation, e.g. sonication, high-speed stirring, etc. The temperature range of at least about 60° C. to at less than about 100° C. is most preferred. Further, it is generally preferable, although not required, that the aqueous solution be heated to a temperature lower than its boiling point to prevent significant evaporation of the aqueous solvent during dissolution. Dissolution temperatures of 100° C. or higher that do not boil off the aqueous solvent can be obtained by means known to those of skill in the art, such as by increasing the atmospheric pressure that the solution is subjected to during heating. One common means of achieving this result is by autoclaving the solution.

Stable baclofen solutions can be produced by acidification and back titration. Baclofen solutions having concentrations up to about 10.0 mg/mL can be prepared by dissolving baclofen in an acidic solution, preferably one having a pH lower than the pKa₁ of baclofen. For example, pH values lower than about 3.87, lower than about 3.0, lower than about 2.0, lower than about 1.5, or even lower than a pH of about 1.0 can be used advantageously. Surprisingly, once the baclofen has been dissolved in the acidic solution, and prior to pharmaceutical administration, the baclofen solution can be back titrated to a pH of 4.0 to 8.5 without precipitation of baclofen particulates. The titration is carried out by adding a base to the acidic solution until the pH is adjusted to a pH in the desired range. A final pH of 5.0 to 7.0 is currently preferred for baclofen solutions intended for pharmaceutical uses such as intrathecal injection, but pH ranges of 4.5 to 8.0 and of 4.0 to 8.5 can also be suitable for such uses. These pH ranges are intended to be illustrative of appropriate values for uses such as intrathecal injection. The appropriate pH ranges for any particular pharmaceutical application will be readily apparent to those skilled in the art, and the final pH of the baclofen solution can be any pharmaceutically acceptable pH appropriate for a given use. In addition, baclofen solutions prepared by this method can be stored at a pH that is not appropriate for a given pharmaceutical use so long as the solution is titrated to a pharmaceutically acceptable pH prior to administration.

Alternately, stable baclofen solutions can be produced by alkalinization and back titration. That is, solutions having concentrations of baclofen of about 10.0 mg/mL or lower can be prepared by dissolving baclofen in a basic solution, preferably one having a pH higher than the pKa₂ of baclofen. For example, solutions of pH higher than about 9.62, higher than about 10.0, higher than about 11.0, higher than about 12.0, and even higher than the pH of about 13.0 can be used advantageously. Once the baclofen is dissolved in the basic solution the pH can be back titrated to a pH of about 4.0 to 8.5, or preferably can be titrated to a pH of 5.0 to 7.0, or to other pH values appropriate for pharmaceutical uses such as intrathecal injection, as discussed above. For use in other applications, pharmaceutical or otherwise, or during storage prior to use the baclofen solution can be titrated to a lower pH or can be maintained for some period of time at the original basic pH.

Presently, intrathecal baclofen is stored in ampoules. Typical procedure for filling infusion systems includes breaking the ampoules to open them, removing the drug from the ampoule and filtering it using a syringe with an in-line filter and needle, removing the needle from the syringe and replacing it with a catheter that includes a second in-line filter and needle. The needle is then inserted through the skin into the implanted pump reservoir and the fluid is dispensed and filtered, filling the infusion system reservoir with the drug. This process may need to be done from 1 to 4 times for a single filling, depending on the reservoir size and the ampoule configuration selected. There are multiple issues with the current process and the need to enhance safety with intrathecal drugs is paramount.
US 2005/0004219 A1 discloses a system including a reservoir, a pump coupled to the reservoir, a catheter coupled to the pump and adapted for delivering a therapeutic agent to a cerebrospinal fluid of a patient; and an injectable gabapentin composition housed in the reservoir and deliverable through the catheter.
US 2010/0106097 A1 is directed to an indicator strip on a syringe that is used to test compatibility of fluid within a syringe before injecting the fluid into a medical device.
The present invention relates to a drug delivery system comprising: a pre-filled syringe containing a sterile solution consisting of baclofen in a sodium chloride solution, wherein said solution is suitable for intrathecal delivery, and wherein said pre-filled syringe is packaged, labeled, and sterilized.

As used herein, the terms below have the meanings indicated.

The term "pre-filled," as used herein, means containing an exact, predetermined dose of a sterile pharmaceutical composition.

The present invention implements a pre-filled syringe that is ready for immediate delivery to the infusion system. The packaging system includes a syringe with a leur-lock tip filled with intrathecal baclofen, a color coding system (label) for the various concentrations of the drug product and size of syringe, a package, a label, and instructions for use.

Since the drug in the syringe is already prepared, the process of drawing up and filtering the drug into a syringe prior to refilling the infusion system is eliminated. Eliminating this process makes filling and refilling the infusion system safer and easier. The pre-filled syringe is easier to use because the practitioner does not have to draw up and filter the drug while administering the therapy to the patient. The syringe's label or plunger is color coded by concentration and syringe sizes, thereby reducing practitioner error and increasing safety to the recipient. Higher concentration formulations will be available to reduce the number of times the recipient must be injected with the needle. The pre-filled syringe also eliminates the potential of contamination of the drug with glass particles from the ampoule, bacteria and the like.

Further features and advantages of the invention, as well as the structure and operation of various embodiments of the invention, are described in detail below.

### BRIEF DESCRIPTION OF THE DRAWINGS

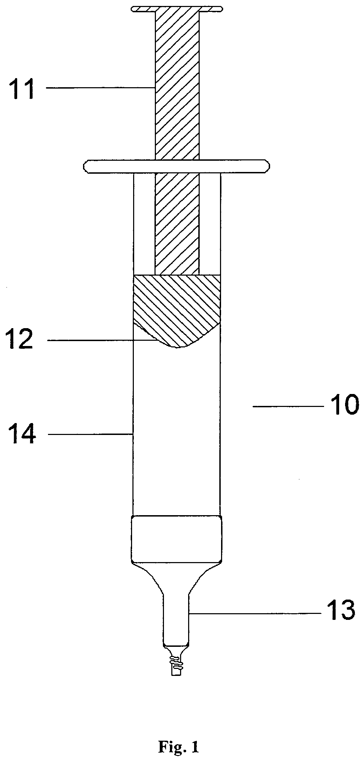

Figure 1 is an illustration of the pre-filled syringe according to an exemplary embodiment.
Figure 2 is an illustration of the pre-filled syringe as used with an infusion system.
Referring to Figure 1, the pre-filled syringe 10 comprises a barrel 14, a plunger 11 with attached gasket 12, and a leur-lock tip 13.

The barrel 14 is made of glass or plastic having two open ends. The pre-filled syringe 10 can have sizes of 5 milliliters, 20 milliliters, or 40 milliliters. One end of the barrel 14 is closed off by a plunger 11 that forces the medical liquid (not shown) to the other end of the barrel 14 when dispensing. A gasket 12 is attached to the plunger 11 for sealing the medical liquid in the barrel 14. The gasket 12 is made of a rubbery elastic material, such as natural rubber or synthetic rubber. The dispensing end of the barrel 14 is closed off by a leur-lock tip 13. The leur-lock tip 13 mates with the infusion system for dispensing the medical liquid.

The pre-filled syringe 10 is filled with a medical fluid, in particular, intrathecal baclofen. The solution comprises baclofen USP, Sodium Chloride, and water and is approved by the Food and Drug Administration. Other solutions besides saline solutions that are suitable for intrathecal delivery may be used in the present invention as well. The solution is formed aseptically, is terminally sterilized, and inserted into sterilized syringes. The concentrations of intrathecal baclofen can vary between about 0.5 mg/mL and about 8.0 mg/mL. Preferred concentrations of intrathecal baclofen are 0.5 mg/mL, 2.0 mg/mL, 3.0 mg/mL, and 4.0 mg/mL. The available high concentrations and large syringe sizes eliminate the need for multiple operations to fill the pump reservoir, thus reducing the potential of practitioner error and thereby increasing the safety of the recipient. Further, the syringes have minimal head space, which leads to a decrease in degradation of the baclofen solution via oxidation. The label or plunger 11 of each pre-filled syringe 10 has a distinct color for identifying the dosages. The color-coded system further helps to eliminate practitioner error of injecting the wrong dose. The product is packaged, labeled, and sterilized.

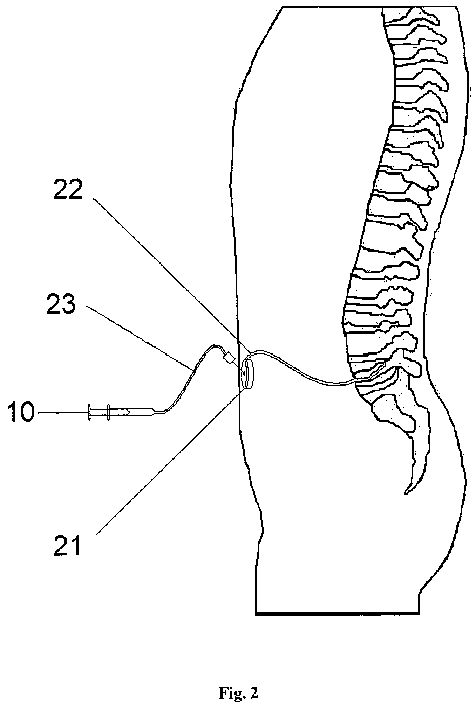

Figure 2 displays the pre-filled syringe 10 as used with the pump system. Pump refill kits are commercially available from Medtronic^{®} and include a catheter 23 for connecting the pre-filled syringe to the pump 21. Intrathecal baclofen may be dispensed from the pre-filled syringe 10, through the catheter 23, into the pump 21 without the baclofen being drawn and filtered. The pump 21 then pumps the intrathecal baclofen through a second catheter 22 to a desired location in the body. The pre-filled syringe can be used with the Medtronic SynchroMed Infusion System^{®}, the Johnson and Johnson Codman^{®} division pumps, and InSet^{®} technologies pumps.

In another embodiment, the vial described herein is coated with a compound that deactivates the glass surface, so possible reactions between baclofen and the glass are eliminated. Possible vials with this coating include vials treated with SCHOTT Type I plus^{®} coating technology.

In a further embodiment, the vial described herein is silanized to prevent adsorption and precipitation of baclofen.

The presence of oxygen may lead to the oxidation of baclofen. In order to reduce the chances of oxidation of the baclofen solution while in vials, a blanket of nitrogen gas is laid across the vials before they are sealed to displace any oxygen present. Oxidation of the baclofen solution in syringes is minimized by the lack of head space in the syringes, which limits the presence of any gases, including oxygen, within the syringe.

In yet another embodiment, the baclofen solution is stored under a nitrogen atmosphere within the vial.

## Claims

1. An intrathecal drug delivery system comprising:
(a) a sterilised syringe (10) with a leur-lock tip (13); **characterised in that**
(b) the sterilised syringe containing a sterile, stable solution consisting of baclofen, sodium chloride and water which has been formed aseptically and terminally sterilized and inserted into the sterilized syringe with minimal head space;
(c) a color coding system on a label or plunger of the sterilised syringe indicative of various concentrations of baclofen of (b) or indicative of size of the sterilised syringe; and
(d) instructions for use.

2. The intrathecal drug delivery system of claim 1, wherein said sterile solution of intrathecal baclofen has a baclofen concentration between 0.5 mg/mL and 8.0 mg/mL.

3. The intrathecal drug delivery system of claim 1, wherein said sterile solution of intrathecal baclofen has a baclofen concentration between 0.5 mg/mL and 4.0 mg/mL.

4. The intrathecal drug delivery system of claim 1, wherein said sterile solution of intrathecal baclofen has a baclofen concentration of 3.0 mg/mL.

5. The intrathecal drug delivery system of claim 1, wherein said sterile solution of intrathecal baclofen has a baclofen concentration of 2.0 mg/mL.

6. The intrathecal drug delivery system of claim 1, wherein said sterile solution of intrathecal baclofen has a baclofen concentration of 0.5 mg/mL.

7. The intrathecal drug delivery system of any of claims 1-6, wherein said pre-filled syringe contains 5 mL or greater of said sterile solution of intrathecal baclofen.

8. The intrathecal drug delivery system of any of claims 1-6, wherein said pre-filled syringe contains 20 mL or greater of said sterile solution of intrathecal baclofen.

9. The intrathecal drug delivery system of any of claims 1-6, wherein said pre-filled syringe contains 40 mL of said sterile solution of intrathecal baclofen.

## Patentansprüche

1. System zur intrathekalen Arzneimittelabgabe, umfassend:
(a) eine sterilisierte Spritze (10) mit einer Leur-Lock-Spitze (13); **dadurch gekennzeichnet, dass**
(b) die sterilisierte Spritze eine sterile, stabile Lösung enthält, die aus Baclofen, Natriumchlorid und Wasser besteht, die aseptisch gebildet und abschließend sterilisiert und mit minimalem Kopfraum in die sterilisierte Spritze eingeführt wurde;
(c) ein Farbcodierungssystem auf einem Etikett oder Kolben der sterilisierten Spritze verschiedene Konzentrationen von Baclofen von (b) angibt oder die Größe der sterilisierten Spritze angibt; und
(d) eine Gebrauchsanweisung.

2. System zur intrathekalen Arzneimittelabgabe nach Anspruch 1, wobei die sterile Lösung von intrathekalem Baclofen eine Baclofenkonzentration zwischen 0,5 mg/ml und 8,0 mg/ml aufweist.

3. System zur intrathekalen Arzneimittelabgabe nach Anspruch 1, wobei die sterile Lösung von intrathekalem Baclofen eine Baclofenkonzentration zwischen 0,5 mg/ml und 4,0 mg/ml aufweist.

4. System zur intrathekalen Arzneimittelabgabe nach Anspruch 1, wobei die sterile Lösung von intrathekalem Baclofen eine Baclofenkonzentration von 3,0 mg/ml aufweist.

5. Intrathekales Arzneimittelabgabesystem nach Anspruch 1, wobei die sterile Lösung von intrathekalem Baclofen eine Baclofenkonzentration von 2,0 mg/ml aufweist.

6. System zur intrathekalen Arzneimittelabgabe nach Anspruch 1, wobei die sterile Lösung von intrathekalem Baclofen eine Baclofenkonzentration von 0,5 mg/ml aufweist.

7. System zur intrathekalen Arzneimittelabgabe nach einem der Ansprüche 1-6, wobei die vorgefüllte Spritze 5 ml oder mehr der sterilen Lösung von intrathekalem Baclofen enthält.

8. System zur intrathekalen Arzneimittelabgabe nach einem der Ansprüche 1-6, wobei die vorgefüllte Spritze 20 ml oder mehr der sterilen Lösung von intrathekalem Baclofen enthält.

9. System zur intrathekalen Arzneimittelabgabe nach einem der Ansprüche 1-6, wobei die vorgefüllte Spritze 40 ml der sterilen Lösung von intrathekalem Baclofen enthält.

## Revendications

1. Système d'administration de médicament intrathécal comprenant :
(a) une seringue stérilisée (10) dotée d'une pointe à verrouillage Luer (13) ; **caractérisé en ce que**
(b) la seringue stérilisée contient une solution stérile stable constituée de baclophène, de chlorure de sodium et d'eau qui a été formée de façon aseptique et stérilisée de manière terminale et insérée dans la seringue stérilisée avec un espace de tête minimal ;
(c) un système de codage par couleurs sur une étiquette ou le piston de la seringue stérilisée indiquant diverses concentrations de baclophène de (b) ou indiquant la taille de la seringue stérilisée ; et
(d) des instructions d'utilisation.

2. Système d'administration de médicament intrathécal selon la revendication 1, ladite solution stérile de baclophène intrathécal comportant une concentration de baclophène comprise entre 0,5 mg/ml et 8,0 mg/ml.

3. Système d'administration de médicament intrathécal selon la revendication 1, ladite solution stérile de baclophène intrathécal comportant une concentration de baclophène comprise entre 0,5 mg/ml et 4,0 mg/ml.

4. Système d'administration de médicament intrathécal selon la revendication 1, ladite solution stérile de baclophène intrathécal comportant une concentration de baclophène de 3,0 mg/ml.

5. Système d'administration de médicament intrathécal selon la revendication 1, ladite solution stérile de baclophène intrathécal comportant une concentration de baclophène de 2,0 mg/ml.

6. Système d'administration de médicament intrathécal selon la revendication 1, ladite solution stérile de baclophène intrathécal comportant une concentration de baclophène de 0,5 mg/ml.

7. Système d'administration de médicament intrathécal selon l'une quelconque des revendications 1 à 6, ladite seringue préremplie contenant 5 ml ou plus de ladite solution stérile de baclophène intrathécal.

8. Système d'administration de médicament intrathécal selon l'une quelconque des revendications 1 à 6, ladite seringue préremplie contenant 20 ml ou plus de ladite solution stérile de baclophène intrathécal.

9. Système d'administration de médicament intrathécal selon l'une quelconque des revendications 1 à 6, ladite seringue préremplie contenant 40 ml de ladite solution stérile de baclophène intrathécal.
